# EUROPÄISCHE PATENTSCHRIFT

(11) **EP 2 328 483 B1**
(45) Veröffentlichungstag und Bekanntmachung des Hinweises auf die Patenterteilung: **11.01.2017**
(21) Anmeldenummer: 09813955.3
(22) Anmeldetag: 18.09.2009
(51) Int. Cl.: A61B 17/00, A61M 5/315, B05C 17/005, B01F 13/00, B01F 15/02, B01F 5/06

(54) **ANSCHLUSSSTÜCK MIT MISCHELEMENT FÜR AUSTRAGANORDNUNG**
CONNECTOR HAVING MIXING ELEMENT FOR DISCHARGE ARRANGEMENT
RACCORD DOTÉ D'UN ÉLÉMENT DE MÉLANGE POUR SYSTÈME D'EXTRACTION

(30) Priorität: 22.09.2008 CH 14952008
(43) Veröffentlichungstag der Anmeldung: 08.06.2011
(73) Patentinhaber: Medmix Systems AG, 6343 Rotkreuz (CH)
(72) Erfinder: GRETER, Andy, CH-6340 Baar (CH); KAYSER, Ralph Egon, CH-6004 Luzern (CH); KELLER, Wilhelm A., CH-6402 Merlischachen (CH)
(74) Vertreter: Detken, Andreas
(86) Internationale Anmeldenummer: PCT/CH2009/000307
(87) Internationale Veröffentlichungsnummer: WO 2010/031197

(56) Entgegenhaltungen:
- EP-A- 0 689 874
- EP-A- 1 825 927
- WO-A-00/21842
- WO-A-94/14698
- WO-A1-01/66017
- WO-A1-95/31137
- DE-A1- 19 618 693
- US-A- 6 161 730
- US-A1- 2006 280 690

## Beschreibung

### Technisches Gebiet

Die vorliegende Erfindung betrifft eine Austraganordnung zum Austragen einer Mischung aus wenigstens zwei Komponenten, insbesondere einer Austraganordnung mit einem Anschlussstück zur Befestigung von Zubehörteilen für die Austraganordnung.

### Stand der Technik

Aus dem Stand der Technik sind zahlreiche Austraganordnungen zum Austragen von Mischungen z. B. aus Doppelspritzen oder Doppelkartuschen bekannt, bei welchen in den jeweiligen Behältern der Doppelspritze je eine Mischkomponente vorgesehen ist, die mittels der Austraganordnung erst kurz vor ihrer Anwendung gemischt werden. Beispielsweise ist bei Medikamenten, die in gemischter Form nicht stabil sind, oder bei Mischungen, die nach dem Mischen in kurzer Zeit aushärten, ein Mischen erst kurz vor der Anwendung erforderlich. Insbesondere im Bereich der Medizinaltechnik ist es bei vielen Anwendungen notwendig, eine Mischung erst kurz vor ihrer Applikation, bzw. ihrer Injektion, herzustellen. Die Mischkomponenten werden in der Regel in den einzelnen Behältern der Doppelspritze gelagert oder können erst vor der Applikation in die Spritze eingesaugt werden. Auf die Austraganordnung wird in der Regel ein Mischer aufgesetzt, der Einlasskanäle für die jeweilige Komponente aus einem Behälter der Doppelspritze und einen Mischkanal aufweist, in welchen die Einlasskanäle münden. In dem Mischkanal sind Mischelemente in Form einer Wendel oder sonstigen Mischmitteln vorgesehen, so dass beim Austragen der Mischkomponenten aus der Doppelspritze die Komponenten in dem Mischkanal zusammengeführt werden und durch die Mischelemente homogen vermischt werden.

Aus der WO 94/14698 ist beispielsweise ein Austrag- und Mischgerät bekannt, bei dem über den Auslässen der Behälter einer Doppelkartusche ein Mischeraufsatz befestigt werden kann. Der Mischeraufsatz weist an einem Ende Befestigungsflügel auf, die in Befestigungsrillen an der Doppelkartusche eingreifen. Der Mischeraufsatz umfasst ferner zwei Einlasskanäle, die sich in an der Doppelkartusche befestigtem Zustand an die Auslässe der beiden Behälter anschliessen. Die Einlasskanäle münden in einen Mischkanal, der durch ein Rohr gebildet wird, in dem Mischelemente angeordnet sind. Das Rohr weist einen zylindrischen Durchmesser über die gesamte Länge auf und weist vier in Längsrichtung verlaufende Stützstreben auf, die das Rohr an der Befestigungsbasis abstützen.

EP 1 825 927 zeigt eine Mehrkomponentenspritze mit zwei Behältern für je eine Komponente mit einem Mischerauslass. Der Mischerauslass ist als Verlängerung der Behälter in Form eines Rohres ausgebildet, in dem ein Mischelement untergebracht ist. Das Mischrohr mündet derart ins Innere der Mehrkomponentenspritze, dass Öffnungen in dem Rohr einen Zugang zu jeweils einem Behälter bilden. Beim Austragen der Komponenten aus der Mehrkomponentenspritze werden die einzelnen Komponenten somit unmittelbar aus dem Behälter in das Mischrohr ausgetragen und über die Länge des Mischrohres durch die Mischelemente gemischt.

Die WO 00/21842 zeigt eine Doppelspritze, auf der ein Mischer abnehmbar befestigt werden kann. Der Mischer weist ein Mischrohr auf, das sich konusartig bis zu einem Auslass verjüngt. In dem Mischrohr sind Mischelemente in Form einer Wendel vorgesehen, die sich circa bis zur Hälfte im Inneren des Mischrohres erstreckt. Anschliessend an den Bereich mit dem Mischelement durchläuft die Mischung eine etwa ebenso lange Leerstrecke im Mischrohr, bis das Rohr derart verjüngt ist, dass es zum Austragen der Mischung geeignet ist. Es ist nicht vorgesehen, ein Zubehörteil auf das Mischrohr aufzustecken.

Bei den Austraganordnungen zum Austragen einer Mischung nach dem Stand der Technik ist der Mischer entweder integraler Bestandteil der Austraganordnung oder kann als eigenständiges Teil auf der Austraganordnung angebracht werden. Sofern auf den Mischer weitere Zubehörteile aufgesetzt werden, verlängert sich der Weg, den eine Mischung bis zum Austragen innerhalb der Austraganordnung durchlaufen muss, so dass sich das Verlustvolumen, der in der Austraganordnung verbleibenden Mischung vergrössert und die Zeit, bis die Mischung ausgetragen ist, verlängert. Beides kann sich nachteilig auf die Mischung auswirken. Kurze Verweilzeiten und ein kleines Verlustvolumen sind jedoch für viele Substanzen von grosser Bedeutung. Im Fall von aushärtenden Mischungen muss die Verweilzeit im Mischer kleiner sein als die Reaktionszeit der Komponenten, da sonst der Mischer verblockt.

### Darstellung der Erfindung

Es ist daher eine Aufgabe der vorliegenden Erfindung, eine Austraganordnung mit einer Mischeinrichtung vorzusehen, die geeignet ist, Zubehörteile der Austraganordnung aufzunehmen, ohne dass das Verlustvolumen nachteilig beeinflusst wird oder die Verweilzeit der Mischung innerhalb der Austraganordnung unnötig verlängert wird.

Diese Aufgabe wird durch eine Austraganordnung mit den Merkmalen des Anspruchs 1 gelöst. Vorteilhafte Ausgestaltungen sind in den abhängigen Ansprüchen angegeben.

Es wird also eine Austraganordnung vorgeschlagen, die ein Anschlussstück zum Anschluss eines Zubehörteils aufweist, mit mindestens einem ersten und einem zweiten Einlasskanal und mit einem Mischkanal, in den die Einlasskanäle münden und der an einem Auslass endet. Ausserdem umfasst die Austraganordnung eine statische Mischvorrichtung mit einem Mischelement, das im Mischkanal angeordnet ist (bevorzugt ein wendelartiges, auf mehrfacher Scherung basierendes Element, z.B. eine Mischwendel, mehrere hintereinander angeordnete Mischwendeln oder eine andere Anordnung von mehreren entlang der Längsrichtung hintereinander angeordneten Klingen, Scherblenden oder Vorsprüngen, die dazu dienen, die Komponenten durch Scherwirkung zu vermischen). Das Anschlussstück weist einen als Befestigungsbereich für das Zubehörteil ausgebildeten Konusbereich auf, dessen Aussenumfang sich entlang einer Längsrichtung zum Auslass des Mischkanals hin kontinuierlich konisch zulaufend verjüngt und der zumindest einen am Auslass endenden distalen Endabschnitt des Mischkanals radial umgibt. Mindestens ein Teil des Mischelements ist dann im vom Konusbereich umgebenen distalen Endbereich des Mischkanals angeordnet, d.h. das Mischelement der Mischvorrichtung erstreckt sich in den distalen Endabschnitt des Mischkanals hinein.

Das Mischelement liegt also radial gesehen innerhalb des Konusbereichs. Die Strecke zum Austragen einer Mischung wird also nicht durch den Befestigungsbereich für das Zubehörteil verlängert, sondern der Befestigungsbereich liegt bezüglich der Längsrichtung "neben" dem Mischbereich mit dem Mischelement. Auf diese Weise wird das Totvolumen im Anschlussstück reduziert. Bevorzugt sind die Mischvorrichtung und der Konusbereich dabei konzentrisch bezüglich der Längsachse des Konusbereichs angeordnet.

Bevorzugt erstreckt sich dabei das Mischelement über mindestens 90% der Länge, besonders bevorzugt im Wesentlichen über die gesamte Länge des vom Konusbereich umgebenen distalen Endabschnitts des Mischkanals, d.h. im Wesentlichen bis zum Auslass des Mischkanals, um das Totvolumen auf ein Minimum zu reduzieren.

Der Konusbereich ist als männlicher Luer-Konus mit einer Steigung der Aussenseite von ca. 6% zur Längsrichtung ausgebildet. Weitere Merkmale eines Luer-Konus können der einschlägigen Norm ISO 594/1-1986 (First Edition, 1986-06-15) entnommen werden, auf die hiermit verwiesen wird und die hiermit durch Verweis in ihrer Gesamtheit in die vorliegende Offenbarung aufgenommen wird. Insbesondere beträgt die Länge des Konusbereichs vorzugsweise mindestens 7.5 mm. Der Durchmesser am distalen Ende des Konusbereichs (d.h. am Auslass) beträgt vorzugsweise 3.92-4.027 mm, der Durchmesser am anderen Ende des Konusbereichs vorzugsweise 4.270-4.315 mm. Indem der Konusbereich einen Luer-Konus bildet, können Zubehörteile über eine genormte Verbindung auf dem Anschlussstück aufgesteckt werden.

Die Austragvorrichtung kann ausserdem umfassen:
wenigstens zwei getrennt oder einstückig ausgebildete Behälter zur Aufnahme je einer Mischkomponente, wobei jeder der Behälter einen Behälterauslass aufweist; und
eine Austrageinrichtung zum Austragen der Mischkomponenten durch die Behälterauslässe.

Das Anschlussstück schliesst sich dann an die Behälterauslässe an.

Die wenigstens zwei Behälter zur Aufnahme je einer Mischkomponente können z. B. in Form einer Doppelspritze oder Doppelkartusche vorliegen. Es ist jedoch auch möglich, zwei einzelne Spritzen an je einem Einlasskanal des Anschlussstücks vorzusehen. Grundsätzlich können auch mehr als zwei Behälter vorgesehen werden. Beispielsweise können drei Behälter, die in einem Dreieck angeordnet sind, vorgesehen werden. Es sind auch mehr als drei Behälter möglich. Die Austrageinrichtung wird beispielsweise durch eine Antriebsstange, wie etwa einen Stössel oder eine Kolbenstange, in jedem Behälter vorgesehen. Dabei können die einzelnen Antriebsstangen in den jeweiligen Behälter an ihrem aus den Behältern herausragenden Ende verbunden sein oder auch durch ein gesondertes Verbindungselement miteinander verbunden werden. Durch das Einschieben der Austrageinrichtung in die Behälter werden die Mischkomponenten durch die Behälterauslässe ausgetragen.

Das Anschlussstück, das sich an die Behälterauslässe anschliesst, ist vorzugsweise von den Auslässen lösbar vorgesehen. Grundsätzlich kann das Anschlussstück aber auch eine feste Verbindung mit den Behälterauslässen eingehen. Vorzugsweise weist das Anschlussstück für jeden Behälterauslass eine Öffnung auf, in welche der Behälterauslass, der z. B. durch einen röhrenartigen Vorsatz an dem Behälter vorgesehen ist, eingeführt werden kann. Grundsätzlich ist es aber auch möglich, das Anschlussstück auch über Gehäuseteilen der Behältergehäuse vorzusehen. Das Anschlussstück kann dann vorteilhafterweise als Halterung für die Behälter genutzt werden.

An die Öffnungen zur Aufnahme der Behälterauslässe schliessen sich die Einlasskanäle des Anschlussstücks an, so dass eine Mischkomponente, die durch den Behälterauslass ausgetragen wird, in den Einlasskanal des Anschlussstücks eingeführt wird. Die einzelnen Einlasskanäle der verschiedenen Behälter münden in den Mischkanal.

Die statische Mischvorrichtung in dem Mischkanal kann verschiedene Mischmittel oder Mischelemente enthalten, wie etwa Wendelelemente oder Scherblenden/Scherblätter. Die Mischvorrichtung kann auch anders gestaltet sein, solange eine ausreichende Mischung der Mischkomponenten entsteht. Solche Mischer sind grundsätzlich bekannt, einfach in der Herstellung und reichen für die meisten Mischungen zum Vermischen der Komponenten aus.

Gemäss der vorliegenden Erfindung ist der Mischkanal zumindest teilweise in einem Konusbereich (der auch als Konuselement bezeichnet werden kann), in anderen Worten in einem kegelstumpfförmigen Bereich/Element vorgesehen. Der Aussenumfang des Konusbereichs ist an dem Ende, das den Einlasskanälen zugewandt ist, grösser als an einem Auslass des Mischkanals. Demnach verjüngt sich der Aussenumfang in Richtung des Mischkanalauslasses. Dabei verläuft die Verjüngung kontinuierlich über die Länge des Mischkanals. Der im Inneren des Konusbereichs liegende Mischkanal kann röhrenartig, bzw. zylindrisch, ausgebildet sein. Es ist aber auch möglich, dass sich auch der Mischkanal in Richtung des Mischkanalauslasses hin verjüngt. Die Mischvorrichtung ist an die Form des Mischkanals angepasst, so dass der Raum des Mischkanals von der Mischvorrichtung ausgefüllt wird.

Der Aussenumfang des Konusbereichs dient als Befestigungsbereich für ein Zubehörteil der Austraganordnung, wie etwa eine Austragdüse, ein Katheter, oder eine Injektionsnadel. Es können auch andere Zubehörteile angeschlossen werden. Zur Befestigung auf dem Konusbereich weist das Zubehörteil vorzugsweise eine röhrenartige oder kanalartige Öffnung auf, in welche das Anschlussstück mit dem Konusbereich eingeschoben bzw. eingesteckt wird. Vorzugsweise dient die Öffnung als Austragkanal oder mündet in einen Austragkanal für die Mischung und erstreckt sich in Längsrichtung durch das Zubehörteil. Der Durchmesser der Kanalöffnung des Zubehörteils liegt zwischen dem grössten und dem kleinsten Durchmesser des Konusbereichs. Bei einem Aufsetzen des Zubehörteils auf den Konusbereich des Anschlussstücks entsteht eine kraftschlüssige Verbindung für das Zubehörteil. Der Innenumfang der Kanalöffnung des Zubehörteils kann die gleiche konische Verjüngung, wie der Aussenumfang des Konuselements, aufweisen und insbesondere als weiblicher Luerkegel ausgebildet sein.

Bei einer Austraganordnung zum Austragen einer Mischung gemäss der vorliegenden Erfindung kann ein Zubehörteil direkt über dem Mischbereich der Austraganordnung vorgesehen werden. Es ist nicht notwendig, das Zubehörteil in einer Art Serienanordnung an einem Befestigungselement im Anschluss an den Mischbereich zu befestigen, wodurch eine Verlängerung der gesamten Austragstrecke entsteht, die sich nachteilig auf das Verlustvolumen der Kanäle und die Verweilzeit der Mischung im Mischer auswirken kann. Der Befestigungsbereich für die Zubehörteile ist gemäss der vorliegenden Erfindung vorteilhafterweise radial neben dem Mischbereich, bzw. parallel bezogen auf die Längsrichtung zu dem Mischbereich, vorgesehen. Die Kanallänge des Mischkanals kann somit gleichzeitig für die Befestigung der Zubehörteile genutzt werden. Die Verweilzeit der Mischung in den Kanälen der Austraganordnung wird somit verkürzt, ohne dass sich dies nachteilig auf die Vermischung der Mischkomponenten auswirkt.

Bei einer Ausführungsform der vorliegenden Erfindung weist das Anschlussstück einen Einführkanal auf, der sich in Längsrichtung an den Mischkanal anschliesst und in proximaler, dem distalen Endbereich abgewandter Richtung, d.h. in Richtung der Behälter, aus dem Anschlussstück heraus mündet. Der Einfiihrkanal kommt vorzugsweise parallel zu den Einlasskanälen für die Behälterauslässe zu liegen und ist zwischen diesen angeordnet. Dadurch verlängert das Anschlussstück in Längsrichtung der Austragsanordnung nicht unnötig. Die Mischvorrichtung des Anschlussstücks, insbesondere das Mischelement, ist durch diesen Kanal in den Mischkanal einführbar. Hierfür wird eine Mischvorrichtung mit einem Mischelement oder mit Mischelementen gewählt, das z. B. als länglicher Stab ausgebildet sein kann, der zumindest in dem Bereich, der in dem Mischkanal zu liegen kommt ein Mischprofil, z. B. in Form einer Wendel oder von Vorsprüngen, aufweist. Die Mischelemente können somit entsprechend den Anforderungen für die Herstellung einer Mischung unterschiedliche Mischprofile oder Elemente mit unterschiedlichen Profilen haben.

In dem Bereich der Mischvorrichtung, der in dem Einführkanal des Anschlussstücks zu liegen kommt, ist die Mischvorrichtung vorzugsweise zylindrisch oder leicht konisch ausgebildet, so dass sie das Volumen des Kanals ausfüllt. Dieser Teil der Mischvorrichtung dient auch zur Verdrehsicherung oder zur Zentrierung, bzw. Positionierung der Mischelemente der Mischvorrichtung im Anschlussstück und somit im Mischkanal. Er wird im Folgenden als Zentrierzylinder bezeichnet. Die Verdrehsicherung oder die Positionierung kann z. B. durch seitliche Vorsprünge an dem Zentrierzylinder und Nuten im Kanal vorgesehen sein, in welche die seitlichen Vorsprünge eingreifen. Die Mischvorrichtung kann somit in einer gewünschten Orientierung innerhalb des Anschlussstücks, bzw. des Mischkanals vorgesehen werden.

Zwischen dem Zentrierzylinder und dem Mischelement der Mischvorrichtung kann eine Trennwand vorgesehen werden. Ist die Mischvorrichtung in das Anschlussstück eingeführt, kommt die Trennwand in dem Bereich zu liegen, in dem die beiden Einlasskanäle in den Kanal zum Einführen der Mischvorrichtung münden. Durch die Trennwand werden die beiden aus den jeweiligen Einlasskanälen zugeführten Komponenten weiterhin von einander getrennt und kommen erst im Bereich der Mischelemente im Mischkanal miteinander in Kontakt.

In einer weiteren Ausführungsform der vorliegenden Erfindung weist das Anschlussstück am Aussenumfang eine Ringnut auf, in der eine Überwurfmutter mit einem Innengewinde drehbar geführt wird. Vorzugsweise ist das Anschlussstück mit der Ringnut und der Überwurfmutter als Luer-Lock-Verbindung gemäss ISO 594-2:1998 ausgebildet. Ein Zubehörteil, das auf das Anschlussstück aufgesetzt wird, kann am Aussenumfang ein Gewinde aufweisen, das mit dem Gewinde der Überwurfmutter korrespondiert. Dadurch kann das Zubehörteil zusätzlich zu der kraftschlüssigen Befestigung am Konusbereich durch eine Schraubverbindung befestigt werden.

Auch bei dieser Ausführungsform kommen vorteilhafterweise sowohl die kraftschlüssige Verbindung des Konusbereichs mit dem Zubehörteil als auch die Schraubverbindung zwischen der Überwurfmutter und einem Gewinde des Zubehörteils im Mischbereich der Austraganordnung zu liegen. Die Austraganordnung, bzw. die Länge der Transferkanäle für die Mischung und somit die Verweilzeit der Mischung in den Kanälen wird nicht durch den Befestigungsbereich für ein Zubehörteil verlängert. Ferner können durch die nach der Erfindung vorgeschlagenen Befestigungsmittel für die Austraganordnung Standard-Zubehörteile verwendet werden, wie sie aus dem Stand der Technik weitreichend bekannt sind.

### Kurze Beschreibung der Zeichnungen

Bevorzugte Ausführungsformen der Erfindung werden anhand der Zeichnung dargestellt, die in keiner Weise einschränkend auszulegen ist. Aus den Figuren der Zeichnung offenbar werdenden Merkmale sollen als zur Offenbarung der Erfindung gehörend verstanden werden. In der Zeichnung stellen dar:
- Figur 1:: Explosionsdarstellung einer Austraganordnung nach der vorliegenden Erfindung,
- Figur 2:: Austraganordnung nach Figur 1 in zusammengesetztem Zustand,
- Figur 3:: Längsschnitt durch eine Austraganordnung nach Figur 2 und
- Figur 4:: Längsschnitt durch eine Austraganordnung mit Zubehörteil.

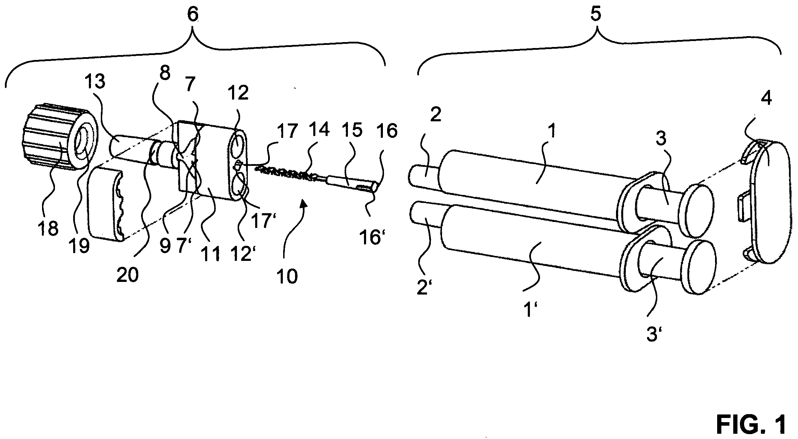

In Figur 1 ist eine Austraganordnung gemäss einer Ausführungsform der vorliegenden Erfindung in einer Explosionsdarstellung gezeigt. Die Austraganordnung weist zwei Spritzen mit jeweils einem Behälter 1 und 1' zur Aufnahme je einer Mischkomponente, je einem Auslass 2 und 2' an einem Ende der Behälter und jeweils eine Antriebsstange 3 und 3' in Form eines Stössels am anderen Ende des Behälters auf. Die Antriebsstange schliesst die Behälter mit einem in Längrichtung der Behälter beweglichen Kolben flüssigkeitsdicht ab. Es ist ein Verbindungselement 4 vorgesehen, welches die Köpfe der Antriebsstange 3 und 3' verbindet, so dass die Antriebsstangen 3 und 3' und das Verbindungselement 4 eine Einheit bilden. Das Verbindungselement 4 weist Schlitze auf, in welche die Köpfe der Antriebsstangen passgenau eingeführt werden könne.

Die Behälterauslässe 2, 2' ragen in Längsrichtung der Behälter 1, 1' röhrenartig von den Behältern ab. Die Auslässe 2 und 2' müssen dabei nicht zentrisch angeordnet sein. Der röhrenartige Fortsatz kann am Aussenumfang zylindrisch ausgebildet sein, ist jedoch vorzugsweise leicht konisch vorgesehen.

Die Einheit aus den beiden Spritzen, die durch das Verbindungselement 4 über ihre Antriebsstangen 3, 3' miteinander verbunden sind, bilden eine Art Doppelspritze 5. Grundsätzlich ist es auch möglich, Doppelspritzen zu verwenden, bei welchen die einzelnen Behälter in einem gemeinsamen Gehäuse vorgesehen sind.

Ferner ist in Figur 1 ein Anschlussstück 6 gezeigt, dass zwei Einlasskanäle 7 und 7', einen Mischkanal 8 und einen Kanal 9 zum Einführen einer Mischvorrichtung 10 in das Anschlussstück umfasst. Die Kanäle 7, 7', 8 und 9 sind in einem Gehäuse 11 des Anschlussstücks untergebracht. Das Gehäuse 11 und das Konuselement 13 bilden eine Einheit. Das Anschlussstück ist für die Austraganordnung gemäss der vorliegenden Ausführungsform für den Anschluss von zwei Mischkomponentenbehältern ausgelegt. Es ist jedoch auch möglich, an dem Gehäuse auch einen dritten oder vierten Einlasskanal oder mehr Einlasskanäle vorzusehen. Das Gehäuse 11 nach der vorliegenden Ausführungsform ist länglich ausgebildet und weist zwei nebeneinander angeordnete Öffnungen 12 und 12' auf, die zur Aufnahme der Behälterauslässe 2 und 2' vorgesehen sind. Die Aufnahmeöffnungen 12 und 12' vertiefen sich röhrenartig in das Innere des Gehäuses 11 und sind parallel zueinander angeordnet. Die Einlasskanäle 7 und 7' beginnen am innen im Gehäuse liegenden Ende der Aufnahmeöffnungen 12 und 12'. Die Aufnahmeöffnungen 12 und 12' können zylindrisch ausgebildet sein, sind jedoch vorzugsweise mit der gleichen konischen Neigung versehen, wie die Auslässe 2 und 2' der Doppelspritze 5. Die Auslässe 2 und 2' werden in die Aufnahmeöffnungen 12 und 12' eingeschoben, so dass eine Fluidverbindung zwischen den Auslässen 2 und 2' der Doppelspritze und den Einlasskanälen 7 und 7' des Anschlussstücks 6 ausgebildet wird. Die Auslässe 2 und 2' gehen dabei mit den Aufnahmeöffnungen 12 und 12' einen Passsitz oder Klemmsitz ein, so dass das Anschlussstück 6 sicher auf der Doppelspritze 5 gehalten wird. Grundsätzlich sind anstelle eines Passsitzes auch andere Verbindungsarten möglich. Beispielsweise kann eine elastische Schnappverbindung vorgesehen sein oder eine Verriegelung, die aktiv manuell betätigt wird.

An dem den Aufnahmeöffnungen 12 und 12' gegenüberliegenden Ende schliesst sich an das Gehäuse 11 des Anschlussstücks 6 ein Konuselement 13 an. Der Mischkanal 8 mündet aus dem Gehäuse 11 in das Konuselement 13 und verläuft durch das Konuselement 13, bis er an dem Ende des Konuselements, das dem Gehäuse 11 gegenüberliegt, austritt. Der Mischkanal 8 verläuft in der dargestellten Ausführungsform zum grössten Teil innerhalb des Konuselements 13. Dies ist vorteilhaft, da dabei die Länge des Konuselements 13 zum einen zum Mischen der Mischkomponenten und zum anderem zur Befestigung eines Zubehörteil für die Austraganordnung, wie nachfolgend beschrieben wird, genutzt werden kann. Grundsätzlich ist es aber auch möglich, den Mischkanal 8 teilweise auch im Gehäuse 11 oder in einem zylindrischen Röhrenbereich vorzusehen.

Der Kanal 9 zum Einführen der Mischvorrichtung 10 in das Gehäuse 11 verläuft zwischen den Aufnahmeöffnungen 12 und 12' und im Wesentlichen parallel zu den Öffnungen 12 und 12', bzw. den Einlasskanälen 7 und 7'. Der Kanal 9 geht in seiner Verlängerung in den Mischkanal 8 über, so dass der Mischkanal 8 und der Kanal 9 im Wesentlichen eine geradlinige Durchführung durch das Anschlussstück 6 bilden. Die Mischvorrichtung 10 kann somit an dem der Doppelspritze 5 zugewandten Ende des Gehäuses 11 in den Kanal 9 eingeführt werden, so dass sie in eingeführten Zustand in den Mischkanal 8 hineinreicht.

Die Mischvorrichtung 10 ist stabförmig ausgebildet und weist an dem Ende, das in dem Mischkanal 8 zu liegen kommt eine Mischwendel 14 auf. An dem gegenüberliegenden Ende ist eine stangenartige Verlängerung als Zentrierzylinder 15 vorgesehen, der an seinem Umfang an sich gegenüberliegenden Seiten Sicherungs- und Positionierflügel 16 und 16' aufweist. Der Zentrierzylinder 15 kann zylindrisch ausgebildet sein, ist in dem gezeigten Beispiel jedoch leicht konisch ausgebildet. Die Mischvorrichtung 10 weist zwischen der Mischwendel 14 und dem Zentrierzylinder 15 eine Trennwand 22 auf Ist die Mischvorrichtung in den Mischkanal 8 und den Kanal 9 eingeführt, kommt die Trennwand in dem Mündungsbereich der beiden Einlasskanäle 7 und 7' zu liegen, in dem die beiden Kanäle 7, 7' in einander einmünden und den Beginn des Mischkanals 8 bilden.

Der Kanal 9 im Gehäuse 11 weist an dem Ende, an welchem die Mischvorrichtung 10 eingeführt wird, in Längsrichtung des Kanals verlaufende Sicherungs- und Positionierrillen 17 und 17' auf. Wird die Mischvorrichtung 10 mit dem Ende der Mischwendel 14 in den Kanal 9 eingeführt, wird sie soweit innerhalb des Kanals 9 vorgeschoben, dass die Mischwendel 14 im Bereich des Konuselements 13 im Mischkanal 8, der Zentrierzylinder 15 innerhalb des Kanals 9 im Gehäuse 11 und die Trennwand 22 im Mündungsbereich der Einlasskanäle 7, 7' zu liegen kommen. Der Zentrierzylinder 15 verschliesst dabei den Kanal 9, so dass die durch die Einlasskanäle 7 und 7' zugeführten Mischkomponenten nicht in den Kanal 9 fliessen könne. Die Sicherungs- und Positionierflügel 16 und 16' des Zentrierzylinders 15 greifen in vollständig in das Gehäuse eingeschobenem Zustand der Mischvorrichtung 10 in die Sicherungs- und Positionierrillen 17 und 17' des Gehäuses ein. Die Flügel und die Rillen dienen zum Einen als Verdrehsicherung, so dass die Mischvorrichtung 10 nicht innerhalb des Gehäuses 11, bzw. des Konuselements 13 drehbar ist. Das Anschlussstück 6 bildet somit einen statischen Mischer beim Abmischen der Mischkomponenten mit der erfindungsgemässen Austraganordnung. Zum Anderen dienen die Flügel und die Rillen dazu, die Mischvorrichtung 10 in einer vorbestimmten Winkelausrichtung im Gehäuse des Anschlussstücks zu positionieren, bzw. zu zentrieren. Die Position der Flügel und Rillen ist derart abgestimmt, dass die Trennwand 22, die beiden Komponenten, die aus den Einlasskanälen 7, 7' von einander trennt bis die Mischwendel 14 beginnt. Die Komponenten aus den Behältern bleiben dadurch solange von einander getrennt, bis mit dem Mischprozess durch die Mischwendel 14 begonnen wird. Ein langsames vermischen am Beginn des Mischkanals, an dem noch kein Mischprofil angeordnet ist, wird dadurch unterbunden.

Grundsätzlich können innerhalb des Gehäuses mit dem Konuselement Mischvorrichtungen mit unterschiedlichen Mischelementen angeordnet werden. Die Mischelemente können sich beispielsweise durch ihre Mischprofile unterscheiden, das heisst anstelle der Mischwendel 14 kann eine Mischblende oder andere Mischprofile vorgesehen werden.

Ferner ist an dem Anschlussstück 6 eine Überwurfmutter 18 vorgesehen. Die Überwurfmutter ist röhrenartig ausgebildet und weist an ihrem Aussenumfang Griffrillen zum Ergreifen der Mutter und an ihrem Innenumfang ein Innengewinde auf. An dem dem Gehäuse 11 zugewandten Ende weist die Überwurfmutter 18 einen nach innen ragenden Ringabsatz 19 auf.

Auf dem Konuselement 13 ist nahe dem Gehäuse 11 eine Ringnut 20 umlaufend vorgesehen. Die Überwurfmutter 18 wird über das Konuselement 13 aufgesetzt, so dass der Ringabsatz 19 innerhalb der Ringnut 20 zu liegen kommt, so dass die Überwurfmutter 18 gegenüber einer Bewegung in Längsrichtung des Konuselements 13 gesichert ist. Die Überwurfmutter 18 ist innerhalb der Ringnut 20 drehbar gelagert.

In der gezeigten Ausführungsform bilden das Konuselement 13 und die Überwurfmutter 18 eine Luer-Lock-Verbindung. An dieser Luer-Lock-Verbindung können verschiedene Zubehörteile der Austraganordnung, wie z. B. eine Austragdüse oder eine Injektionskanüle, welche ebenfalls den Luer-Lock-Mechanismus verwenden, aufgesetzt werden. Bei einer anderen Ausführungsform einer Austragsanordnung nach der Erfindung, ist keine Überwurfmutter notwendig, sondern das Konuselement 13 weist eine derartige Steigung auf, dass eine Luer-Verbindung ausgebildet ist. Auf diese Luer-Verbindung können wiederum Zubehörteile der Austraganordnung mit einer entsprechenden weiblichen Luer-Verbindung aufgesetzt werden. Das Zubehörteil ist dann mittels Kraftschluss zwischen dem Konuselement und dem Zubehörteil auf dem Konuselement, bzw. dem Anschlussstück, befestigt. Es wird somit eine Art Klemmsitz oder Passsitz ausgebildet.

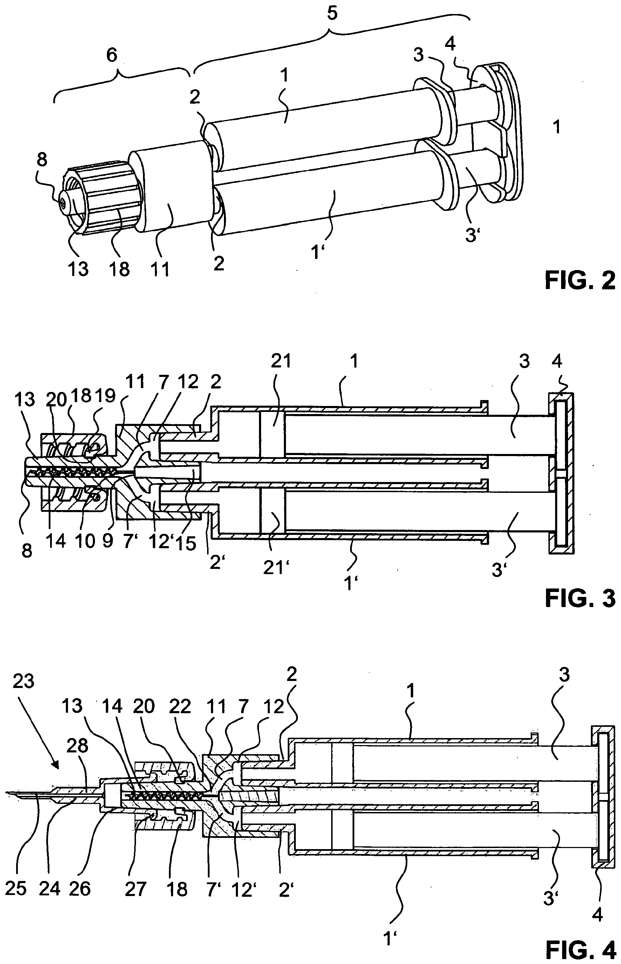

In Figur 2 ist die Austraganordnung gemäss Figur 1 in zusammengesetztem Zustand gezeigt. Das Verbindungselement 4 ist auf die Antriebsstange 3 und 3' aufgesetzt und verbindet diese miteinander. Die Auslässe 2 und 2' der Behälter 1 und 1' sind in die Anschlussöffnungen am Gehäuse 11 des Anschlussstücks 6 eingeschoben. Über dem Konuselement 13 ist die Überwurfmutter 18 angeordnet. Auf die Luer-Lock Verbindung, die durch das Konuselement 13 und die Überwurfmutter 18 ausgebildet wird, kann nun ein Zubehörteil der Austraganordnung aufgesetzt werden, so dass die Austraganordnung funktionsbereit ist.

In Figur 3 ist ein Längsschnitt durch eine Austraganordnung gemäss Figur 2 gezeigt. In den Behältern 1 und 1' sind die Antriebsstangen 3 und 3' aufgenommen an deren in den Behältern liegenden Enden Kolben 21 und 21' vorgesehen sind, welche mit der Innenwand der Behälter 1 und 1' flüssigkeitsdicht abschliessen, aber innerhalb der Behälter verschieblich sind. An dem gegenüberliegenden Ende der Antriebsstangen 3 und 3' ist das Verbindungselement 4 angeordnet und verbindet diese Enden miteinander. Die Auslässe 2 und 2' der Behälter 1 und 1' sind in die Anschlussöffnungen 12 und 12' in dem Gehäuse 11 des Anschlussstücks eingeschoben. Die röhrenartig ausgebildeten Auslässe 2 und 2' gehen mit den Öffnungen 12 und 12' in dem Gehäuse 11 einen Klemmsitz ein, so dass das Anschlussstück 6 fest über den Auslässen 2 und 2' sitzt.

Innerhalb des Mischkanals 8 und des Kanals 9 ist die Mischvorrichtung 10 angeordnet. Dabei kommt die Mischwendel 14 innerhalb des Mischkanals im Inneren des Konuselements 13 zu liegen und die Verlängerung 15 füllt den Kanal 9 aus.

In dem zusammengesetzten Zustand ergibt sich somit eine Fluidverbindung, die im Inneren der Behälter 1 und 1' beginnt, sich über die Auslässe 2 und 2', in die Einlasskanäle 7 und 7' und durch den Mischkanals 8 erstreckt und ggf. durch ein angeschlossenes Zubehörteil aus der Austraganordnung mündet.

Über dem Konuselement 13 ist die Überwurfmutter 18 angeordnet, deren Ringabsatz 19 in die Ringnut 20 des Konuselements 13 eingreift. Über dem Konus 13 kann nun wie bereits beschrieben, ein Zubehörteil der Austraganordnung befestigt werden.

In Figur 4 ist die Austraganordnung mit aufgesetztem Zubehörteil 23 gezeigt. Das Zubehörteil 23 weist einen Kanülenträger 24 und eine Kanüle 25 auf. Der Kanülenträger 24 umfasst einen Zylinderteil 26 mit einer Öffnung, die über das Konuselement 13 des Anschlussstücks 6 geschoben werden kann. Am Aussenumfang weist das Zylinderteil 26 einen Aussengewindebereich 27 auf, der in das Innengewinde der Überwurfmutter 18 eingreift. Durch Drehen der Überwurfmutter 18 wird das Zylinderteil 26 und somit das Zubehörteil 23 auf das Konuselement 13 gezogen und fest gehalten. Aus dem Inneren des Zylinderteils 26 führt ein Austragkanal 28 durch den Kanülenträger 24 und die Kanüle 25. Wie in Figur 4 zu sehen ist, kommt der Befestigungsbereich, in dem das Zubehörteil 23 auf dem Konuselement 13 befestigt wird in radialer Richtung des Konuselements über dem Mischbereich mit der Mischwendel 14 zu liegen, wie es gemäss der Erfindung vorgesehen ist.

Beim Eindrücken der Antriebsstangen 3 und 3' durch Druck auf das Verbindungselement 4 werden die Mischkomponenten aus den Behältern 1 und 1' über ihre jeweiligen Auslässe 2 und 2' in die sich anschliessenden Einlasskanäle 7 und 7' transferiert. Die beiden Mischkomponenten werden in dem Mischkanal 8 zusammengeführt und durch den anhaltenden Druck auf das Verbindungselement 4 entlang der Mischwendel 14 gepresst, wobei eine Durchmischung aufgrund der Mischwendel entsteht und die beiden Mischkomponenten zu einer Mischung vermischt. Am Ende des Mischkanals 8 tritt die Mischung in den Austragkanal 28 des Zubehörteils 23 ein und durch dieses aus der Austraganordnung aus.

Dabei ist es vorteilhaft, dass die Wege welche die Mischkomponenten innerhalb der Austraganordnung zurücklegen müssen, kurz gehalten sind. Vor allem bei kleinen Volumina an Mischkomponenten bzw. einer Mischung sind kurze Wege relevant, um den Verlust der Mischung entlang der Wege zu reduzieren. Es ist daher vorteilhaft, dass die Länge des Konuselements 13 sowohl zur Herstellung der Mischung als auch zur Befestigung von Zubehörteilen verwendet werden kann.

### Bezugszeichenliste

- 1, 1': Behälter
- 2, 2': Auslass
- 3, 3': Antriebsstange / Stössel
- 4: Stösselverbindungselement
- 5: Doppelspritze
- 6: Anschlussstück
- 7, 7': Einlasskanal
- 8: Mischkanal
- 9: Einführkanal
- 10: Mischvorrichtung
- 11: Gehäuse
- 12, 12': Aufnahmeöffnung
- 13: Konuselement
- 14: Mischwendel
- 15: Zentrierhilfe
- 16, 16': Sicherungs- und Positionierflügel
- 17, 17': Sicherungs- und Positionierrillen
- 18: Überwurfmutter
- 19: Ringabsatz
- 20: Ringnut
- 21,21': Kolben
- 22: Trennwand
- 23: Zubehörteil
- 24: Kanülenträger
- 25: Kanüle
- 26: Zylinderteil
- 27: Aussengewindebeich
- 28: Austragkanal

## Patentansprüche

1. Austraganordnung zum Austragen einer Mischung, umfassend
ein Anschlussstück (6) mit mindestens einem ersten und einem zweiten Einlasskanal (7, 7') und mit einem Mischkanal (8), in den die Einlasskanäle münden und der an einem Auslass endet,
und eine statische Mischvorrichtung (10) mit einem Mischelement (14), das im Mischkanal (8) angeordnet ist,
wobei das Anschlussstück (6) einen Konusbereich (13) aufweist, dessen Aussenumfang sich entlang einer Längsrichtung zum Auslass des Mischkanals (8) hin kontinuierlich konisch zulaufend verjüngt und der einen am Auslass endenden distalen Endabschnitt des Mischkanals (8) radial umgibt, und zumindest ein Teil des Mischelements (14) der Mischvorrichtung (10) in dem vom Konusbereich (13) umgebenen distalen Endabschnitt des Mischkanals (8) angeordnet ist,
**dadurch gekennzeichnet, dass** der Konusbereich (13) als Befestigungsbereich für ein Zubehörteil (23) ausgebildet ist, und
dass der Konusbereich (13) als männlicher Luer-Konus mit einer Steigung von ca. 6% zur Längsrichtung ausgebildet ist.

2. Austraganordnung nach Anspruch 1, wobei das Mischelement (14) sich über mindestens 90% der Länge des vom Konusbereich (13) umgebenen distalen Endabschnitts des Mischkanals in diesem distalen Endabschnitt erstreckt.

3. Austraganordnung nach Anspruch 1, wobei das Mischelement (14) sich im Wesentlichen über die gesamte Länge des vom Konusbereich (13) umgebenen distalen Endabschnitts des Mischkanals und im Wesentlichen bis zum Auslass des Mischkanals (8) erstreckt.

4. Austraganordnung nach einem der vorhergehenden Ansprüche, wobei der Konusbereich (13) eine Länge von mindestens 7 mm, einen minimalen Durchmesser von 3.92-4.027 mm und einen maximalen Durchmesser von 4.270-4.315 mm aufweist.

5. Austraganordnung nach einem der vorhergehenden Ansprüche, welche ausserdem umfasst:
wenigstens zwei Behälter (1, 1') zur Aufnahme je einer Mischkomponente, wobei jeder der Behälter einen Behälterauslass (2, 2') aufweist;
eine Austrageinrichtung (3,3') zum Austragen der Mischkomponenten durch die Behälterauslässe (2, 2'),
wobei sich das Anschlussstück (6) an die Behälterauslässe (2, 2') anschliesst.

6. Austraganordnung nach Anspruch 5, wobei das Anschlussstück (6) von den Behälterauslässen (2, 2') abnehmbar ist.

7. Austraganordnung nach Anspruch 5, wobei das Anschlussstück (6) integral mit den Behälterauslässen (2, 2') verbunden ist.

8. Austraganordnung nach einem der vorhergehenden Ansprüche, wobei das Anschlussstück (6) einen Einführkanal (9) aufweist, der sich in Längsrichtung in einer vom distalen Endbereich abgewandten proximalen Richtung an den Mischkanal (8) anschliesst und der in der proximalen Richtung aus dem Anschlussstück (6) mündet, wobei das Mischelement (14) durch den Einführkanal (9) in den Mischkanal (8) einführbar ist.

9. Austraganordnung nach Anspruch 8, wobei der Einführkanal (9) zwischen den Einlasskanälen (7, 7') verläuft.

10. Austraganordnung nach Anspruch 8 oder 9, wobei der Einführkanal (9) eine Verdrehsicherung (16, 16'; 17, 17') für die Mischvorrichtung (10) aufweist.

11. Austraganordnung nach einem der Ansprüche 8 bis 10, wobei der Einführkanal (9) eine Positioniereinrichtung (16, 16'; 17, 17') für die Mischvorrichtung (10) aufweist.

12. Austraganordnung nach einem der vorhergehenden Ansprüche, wobei das Konuselement (13) eine Ringnut am Aussenumfang aufweist, in der eine Überwurfmutter (18) mit einen Innengewinde gehalten ist.

13. Austraganordnung nach Anspruch 12, wobei das Konuselement mit der Überwurfmutter (18) als Luer-Lock-Verbindung ausgebildet ist.

14. Austraganordnung nach einem der vorhergehenden Ansprüche mit einem auf dem Konusbereich (13) angebrachten Zubehörteil (23).

## Claims

1. A discharge device for discharging a mixture, comprising
a connector (6) with at least a first and a second inlet channel (7, 7'), and with a mixing channel (8) into which the inlet channels open and which ends at an outlet,
and a static mixing device (10) with a mixing element (14), which mixing element (14) is arranged in the mixing channel (8),
wherein the connector (6) has a cone area (13) of which the outer circumference tapers continuously in a conical formation along a longitudinal direction as far as the outlet of the mixing channel (8) and which radially surrounds a distal end portion of the mixing channel (8) ending at the outlet, and at least part of the mixing element (14) of the mixing device (10) is arranged in the distal end portion of the mixing channel (8) surrounded by the cone area (13),
**characterized in that** the cone area (13) is designed as a fastening area for an accessory (23), and
**in that** the cone area (13) is designed as a male Luer cone with a taper of approximately 6% with respect to the longitudinal direction.

2. The discharge device as claimed in claim 1, wherein the mixing element (14) extends along at least 90% of the length of the distal end portion of the mixing channel surrounded by the cone area (13) in this distal end portion.

3. The discharge device as claimed in claim 1, wherein the mixing element (14) extends substantially along the entire length of the distal end portion of the mixing channel surrounded by the cone area (13) and substantially as far as the outlet of the mixing channel (8).

4. The discharge device as claimed in one of the preceding claims, wherein the cone area (13) has a length of at least 7 mm, a minimum diameter of 3.92 - 4.027 mm and a maximum diameter of 4.270 -4.315 mm.

5. The discharge device as claimed in one of the preceding claims, additionally comprising:
at least two containers (1, 1'), each receiving a mixing component, wherein each of the containers has a container outlet (2, 2'),
a discharge mechanism (3, 3') for discharging the mixing components through the container outlets (2, 2'),
wherein the connector (6) adjoins the container outlets (2, 2').

6. The discharge device as claimed in claim 5, wherein the connector (6) is removable from the container outlets (2, 2').

7. The discharge device as claimed in claim 5, wherein the connector (6) is integrally connected to the container outlets (2, 2').

8. The discharge device as claimed in one of the preceding claims, wherein the connector (6) has an insertion channel (9) which, in a proximal direction facing away from the distal end area, adjoins the mixing channel (8) along the longitudinal direction, and which opens out from the connector (6) in the proximal direction, wherein the mixing element (14) is adapted to be inserted through the insertion channel (9) into the mixing channel (8).

9. The discharge device as claimed in claim 8, wherein the insertion channel (9) is arranged between the inlet channels (7, 7').

10. The discharge device as claimed in claim 8 or 9, wherein the insertion channel (9) has an anti-twist mechanism (16, 16'; 17, 17') for the mixing device (10).

11. The discharge device as claimed in one of claims 8 through 10, wherein the insertion channel (9) has a positioning mechanism (16, 16'; 17, 17') for the mixing device (10).

12. The discharge device as claimed in one of the preceding claims, wherein the cone element (13) has an annular groove on the outer circumference, in which annular groove a union nut (18) with an internal thread is held.

13. The discharge device as claimed in claim 12, wherein the cone element with the union nut (18) is designed as a Luer lock connection.

14. The discharge device as claimed in one of the preceding claims, with an accessory (23) mounted on the cone area (13).

## Revendications

1. Un ensemble de décharge pour décharger un mélange, comprenant
un raccord (6) ayant au moins un premier et un deuxième canal d'admission (7, 7') et un canal de mélange (8) dans lequel débouchent les canaux d'admission et qui termine au niveau d'une sortie,
et un dispositif statique de mélange (10) ayant un élément de mélange (14) étant disposé dans le canal de mélange (8),
où le raccord (6) présente un domaine conique (13), dont la périphérie extérieure se rétrécit de manière continue en forme conique le long d'une direction longitudinale en direction de la sortie du canal de mélange (8) et qui entoure radialement une section terminale distale du canal de mélange (8), et au moins une partie de l'élément de mélange (14) du dispositif de mélange (10) est disposé dans la section terminale distale du canal de mélange (8) entourée par le domaine conique (13), **caractérisé en ce que** le domaine conique (13) est formé en tant que domaine de fixation pour une pièce d'accessoire (23) et,
que le domaine conique (13) est formé en tant que cône Luer mâle ayant une pente d'environ 6% relatif à la direction longitudinale.

2. Un ensemble de décharge selon la revendication 1, où l'élément de mélange (14) s'étend au moins à travers 90% de la longueur de la section terminale distale du canal de mélange entourée par le domaine conique (13) dans cette section terminale distale.

3. Un ensemble de décharge selon la revendication 1, où l'élément de mélange (14) s'étend essentiellement à travers la longueur entière de la section terminale distale du canal de mélange entourée par le domaine conique (13) et essentiellement jusqu'à la sortie du canal du mélange (8).

4. Un ensemble de décharge selon une des revendications précédentes, où le domaine conique (13) présente une longueur d'au moins 7 mm, un diamètre minimal de 3,92 à 4,027 mm et un diamètre maximal de 4,270 à 4,315 mm.

5. Un ensemble de décharge selon une des revendications précédentes, comprenant un outre :
au moins deux réceptacles (1, 1') pour la réception de respectivement un composant de mélange, où chacun des réceptacles présente une sortie de réceptacle (2, 2'),
un agencement de décharge (3, 3') pour la décharge des composants de mélange à travers la sortie des réceptacles (2, 2'),
où le raccord (6) se raccorde aux sorties de réceptacle (2, 2').

6. Un ensemble de décharge selon la revendication 5, où le raccord (6) est amovible des sorties de réceptacle (2, 2').

7. Un ensemble de décharge selon la revendication 5, où le raccord (6) est intégralement connecté avec les sorties de réceptacles (2, 2').

8. Un ensemble de décharge selon une des revendications précédentes, où le raccord (6) présente un canal d'insertion (9) qui se raccorde au canal de mélange (8) en direction longitudinale dans une direction proximale opposée à la section terminale distale et qui débouche dans la direction proximale à partir du raccord (6), où l'élément de mélange (14) peut être inséré dans le canal de mélange (8) à travers le canal d'insertion (9).

9. Un ensemble de décharge selon la revendication 8, où le canal d'insertion (9) s'étend entre les canaux d'admission (7, 7').

10. Un ensemble de décharge selon les revendications 8 ou 9, où le canal d'insertion (9) présente un dispositif anti-rotation (16, 16', 17, 17') pour le dispositif de mélange (10).

11. Un ensemble de décharge selon une des revendications 8 à 10, où le canal d'insertion (9) présente un dispositif de positionnement (16, 16', 17, 17') pour le dispositif de mélange (10).

12. Un ensemble de décharge selon une des revendications précédentes, où l'élément conique (13) présente une gorge annulaire sur la périphérie extérieure, dans laquelle un écrou de raccordement (18) ayant un filetage femelle est tenu.

13. Un ensemble de décharge selon la revendication 12, où l'élément conique est formé en tant que connexion « Luer-Lock» (18).

14. Un ensemble de décharge selon une des revendications précédentes ayant une pièce d'accessoire (23) montée sur le domaine conique (13).
